# EUROPÄISCHE PATENTSCHRIFT

(11) **EP 2 055 238 B1**
(45) Veröffentlichungstag und Bekanntmachung des Hinweises auf die Patenterteilung: **07.05.2014**
(21) Anmeldenummer: 08167345.1
(22) Anmeldetag: 23.10.2008
(51) Int. Cl.: A61B 17/04, A61B 17/00

(54) **Vorrichtung zum Bewegen eines Fadens eines chirurgischen Nahtinstrumentes**
Device for moving a thread of a surgical sewing instrument
Dispositif destiné à déplacer un fil d'un instrument chirurgical de suture

(30) Priorität: 24.10.2007 DE 102007052195
(43) Veröffentlichungstag der Anmeldung: 06.05.2009
(73) Patentinhaber: Karl Storz GmbH & Co. KG, 78532 Tuttlingen (DE)
(72) Erfinder: Frey, Sebastian, 68753 Waghäusel (DE); Sauer, Michael, 78532 Tuttlingen (DE)
(74) Vertreter: Witte, Weller & Partner Patentanwälte mbB

(56) Entgegenhaltungen:
- US-A- 3 587 587
- US-A- 4 935 027
- US-A- 4 957 498
- US-A- 5 254 126
- US-A1- 2005 283 171
- US-A1- 2007 179 510

## Beschreibung

Die Erfindung betrifft eine Vorrichtung zum Bewegen eines Fadens eines chirurgischen Nahtinstrumentes, bei dem der Faden unmittelbar durch einen Finger einer Hand einer Handhabungsperson bewegbar ist.

Ein derartiges chirurgisches Nahtinstrument ist aus dem Katalog "Arthroskopie, Sportmedizin, Wirbelsäulen-Chirurgie", 2. Ausgabe 1/2005, Seite 106 (Blatt ART-SHF 14A) der Karl Storz GmbH & Co. KG bekannt.

Dieses chirurgische Nahtinstrument weist einen etwa stabförmigen Körper auf, durch den ein chirurgischer Faden von proximal nach distal zu einem distalen Nahtaufsatz führbar ist. Im Körper ist eine Mulde ausgespart, längs der ein Abschnitt des Fadens freiliegend geführt ist.

Wird das chirurgische Nahtinstrument von Hand ergriffen, kommt der Daumen oder ein Zeigefinger so zum Liegen, dass dieser in die Mulde eingreifen kann. Dadurch kann der Faden von einem Finger einer Handhabungsperson, die das chirurgische Nahtinstrument bedient hin- und herbewegt werden, indem der Finger auf den freiliegenden Abschnitt gelegt wird und diesen beispielsweise durch Bewegen des Fingers von proximal nach distal nach distal verschiebt.

Manche chirurgischen Fäden sind aufgrund ihres Aufbaus an der Außenseite sehr glatt, so dass das Hin- und Herbewegen des Fadens erschwert ist. Dies kann noch dadurch erschwert werden, dass die Hand des Chirurgen, die normalerweise von einem Latexhandschuh bedeckt ist, über den Faden rutschen kann.

Aus der US 4,890,615 ist ein Nahtinstrument in Art einer medizinischen Schere bekannt. Dieses Instrument weist einen lang erstreckten Schaft auf, an dessen proximalem Ende zwei relativ zueinander bewegliche Scherengriffe angeordnet sind. Am distalen Ende ist eine ebenfalls bewegbare Nahtvorrichtung angebracht.

An einem der beiden Griffe ist eine Fadenzuführvorrichtung fest montiert, die zwei über ihre Umfangsflächen aneinander laufende Rollen trägt. Zwischen die Rollen kann ein Faden eingefädelt und durch den langen Schaft des Instruments bis zur distalen Nähvorrichtung geführt werden. Um den Faden zu bewegen ist eine der beiden Rollen soweit freiliegend, dass diese mit einem Finger einer Hand, die das scherenartige Instrument ergriffen hat, gedreht werden kann, wodurch dann der Faden hin- und hertransportiert werden kann.

Der Aufbau des Nahtinstrumentes ist sehr kompliziert, weist zahlreiche Einzelteile auf und insbesondere müssen beide Rollen in einem gesondert montierten Fadenzuführaufbau gehalten und gelagert werden. Dadurch entstehen zahlreiche Bakteriennischen, so dass die Reinigung und insbesondere die Sterilisierung äußerst aufwendig ist.

Aus der US-A-4 935 027 ist ein Nahtinstrument mit einer Fadenführung beschrieben. Die Fadenführung ist dabei derart, dass der Faden von einer proximalseitig angeordneten Vorratsrolle kommend mindestens zwischen einer an dieser Vorratsrolle anliegenden weiteren Rolle durch einen Schaft des Instruments bis zu dessen distalem Ende geführt wird, dort umgelenkt und über ein zweites Maulteil dem Schaft wieder zugeführt und am proximalen Ende wieder aus der Vorrichtung abgeführt wird. Auch das Abführen erfolgt derart, dass das abgeführte Ende des Fadens zwischen der Vorratsrolle und einer weiteren Rolle durchgeführt wird.

In einer Ausgestaltung ist vorgesehen, zwei hintereinander angeordnete Rollenpaare mit sich diametral gegenüberstehenden Rollen vorzusehen, wobei von einer fünften endseitigen Vorratsrolle der Faden abgeführt und an einer Seite zwischen zwei Rollen durch und in den Schaft hineingeführt wird. Dementsprechend wird dann der Faden, der am gegenüberliegenden Ende wieder zwischen zwei Rollen geführt wird, seitlich abgeführt. Durch Drehen der äußeren Vorratsrolle, da diese zumindest an zwei weiteren Rollen anliegt, werden diese ebenfalls bewegt sowie der dazwischen gelegte Faden. In einer Ausgestaltung ist vorgesehen, den gesamten Aufbau an drei oder fünf Rollen abnehmbar auszubilden.

Aus der US-A-3 587 587 ist ein scherenartiger Nadelhalter bekannt, zwischen dessen Maulteilen eine Nadel gehalten werden kann. Um der Nadel, die zwischen den Maulteilen gehalten wird, einen Faden zuführen zu können, ist eine Vorrichtung vorgesehen, die seitlich an einen der beiden Scherenarme anklipsbar ist. Von dem Grundkörper steht ein Zapfen vor, auf den eine Rolle aufgebracht ist, auf der wiederum ein Faden aufgewickelt ist. Der Faden kann nunmehr von Hand von der Rolle abgezogen und in die Öse der gehaltenen Nadel eingeführt werden. Beim Nähvorgang selbst kann dann durch die Nadel über die Verbindung mit dem Faden zur Rolle der Faden nach und nach abgezogen werden.

In der US 2007/0179510 A1 ist eine Vorrichtung zum Zuführen eines Fadens bekannt. Der zuzuführende Faden wird mittig durch das gesamte Gerät hindurchgeführt und tritt an einem distalen Ende aus.

In einem proximalen Griffbereich ist ein Endlosband vorhanden, das um zwei Rollen läuft. Dieser Zusammenbau dient dazu, um einen Fadenabschnitt im Innern des Gehäuses mittels des Endlosbandes hin- und herzubewegen. Dazu ist im Gehäuse ein Fenster ausgespart, aus dem ein Abschnitt des umlaufenden Endlosbandes gegenüberliegend zu dem Abschnitt, der mit dem Faden in Kontakt tritt, durch einen Finger zugänglich ist.

Aufgabe der vorliegenden Erfindung ist es, bei einem wesentlich einfacher aufgebauten chirurgischen Nahtinstrument der eingangs genannten Art mit dem stabförmigen Körper eine Möglichkeit zu schaffen, den Fadentransport zu erleichtern.

Erfindungsgemäß wird die Aufgabe durch eine Vorrichtung zum Bewegen eines Fadens eines chirurgischen Instrumentes gelöst, das einen stabförmigen Körper aufweist, durch den ein chirurgischer Faden von proximal nach distal zu einem distalen Nahtaufsatz führbar ist, wobei der Faden in einer Mulde des Körpers freiliegt, so dass der freiliegende Abschnitt des Fadens unmittelbar durch einen Finger einer Hand einer Handhabungsperson ohne Zuhilfenahme einer Rolle bewegbar ist, wobei die Vorrichtung einen Bügel, der an einem Ende eine frei drehbare Rolle trägt und ferner eine Halterung aufweist, über die der Bügel derart an das stabförmige Nahtinstrument lösbar anclipsbar ist, dass die Rolle im Bereich der Mulde des stabförmigen Nahtinstruments auf dem freiliegenden Fadenabschnitt zum Liegen kommt, so dass der Faden durch die Handhabungsperson über die Rolle bewegbar ist.

Diese Ausgestaltung hat den Vorteil, dass die Vorrichtung im Bedarfsfalle an das chirurgische Nahtinstrument angeclipst werden kann.

Ein solcher Bedarfsfall kann beispielsweise vorliegen, wenn ein Faden bewegt werden soll, der eine extrem glatte Oberfläche aufweist. Durch einen einfachen Anclipsvorgang kann die Vorrichtung an das stabförmige Nahtinstrument angebracht werden und zwar derart, dass die Rolle auf einem freiliegenden Abschnitt des Fadens zum Liegen kommt. Durch Drehen der Rolle kann dann der Faden bewegt werden.

So einfach wie die Vorrichtung aufgeclipst werden kann, kann sie auch vom chirurgischen Nahtinstrument wieder abgenommen werden, so dass dadurch die Reinigung wesentlich vereinfacht ist. Im Gegensatz zu dem zuvor erwähnten Stand der Technik kommt man auch nur mit einer einzigen Rolle und nicht mit zwei aneinander liegenden Rollen aus, denn die Bewegung des Fadens erfolgt nur über die eine Rolle des angeclipsten Instrumentes. Die Rolle erleichtert nicht nur die Handhabung sondern auch das Einfädeln des Fadens, denn bei diesem stabförmigen Nahtinstrument ist ein Bereich des Fadens freiliegend, nämlich im Bereich der Mulde, in der normalerweise der Faden über einen Finger unmittelbar bewegt wird. Beim Einfädeln des Fadens beispielsweise von proximal nach distal könnte die Gefahr bestehen, dass der Faden sich im Bereich der Mulde seitlich aus dem Gerät heraus bewegt. Bei angeclipster Vorrichtung dient diese zusätzlich noch als eine Einführhilfe.

Bei Fadenmaterialien, die eine relativ raue Oberfläche aufweisen oder bei Handhabungspersonen, die auch durch den Handschuh hindurch ein gutes Gefühl zum Bewegen des Fadens haben, kann die Vorrichtung weggelassen werden. Bei anderen, insbesondere glatten Materialien oder wenn aufgrund der Operation Feuchtigkeit auf den Faden gelangt, kann die Vorrichtung einfach aufgeclipst werden und der Bewegungsvorgang dann durch die Rolle der aufgeclipsten Vorrichtung gesteuert werden.

Dies ergibt einen wesentlich einfacheren Aufbau als die eingangs erwähnten fest installierten Rollenmechanismen, die zumindest zwei Rollen benötigen. Die Erfindung erlaubt eine wesentlich höhere Flexibilität gegenüber der Handhabungsperson und die Bauteile sind auch wesentlich einfacher zu reinigen. Es ist beispielsweise auch möglich, die Vorrichtung aus einfachen Kunststoffmaterialien als Wegwerfteil herzustellen, so dass ein Reinigungs- und Sterilisiervorgang vollständig unterbleiben kann.

Diese Ausgestaltung zeigt die hohe Flexibilität der erfindungsgemäßen Ausgestaltung.

In einer weiteren Ausgestaltung der Erfindung ist die Rolle abnehmbar am Bügel angebracht.

Diese Maßnahme hat den Vorteil, dass die Reinigung der Vorrichtung wesentlich vereinfacht ist. Dies eröffnet auch die Möglichkeit, in ein und dieselbe Vorrichtung unterschiedliche Rollen einzusetzen, beispielsweise mit unterschiedlichen Belägen auf der Umfangsseite, um individuell auf besondere ausgestaltete Fäden abstimmen zu können.

In einer weiteren Ausgestaltung der Erfindung ist die Rolle von zwei Haltearmen des Bügels getragen.

Diese Maßnahme hat den Vorteil, dass durch konstruktiv einfache Maßnahmen die Rolle am Bügel gehalten ist. Dies erleichtert nicht nur das Erbauen der Vorrichtung sondern auch später das Reinigen.

In einer weiteren Ausgestaltung der Erfindung ist die Rolle in den Bügel einclipsbar.

Diese Maßnahme hat den Vorteil, dass die Rolle durch einen einfachen Einclipsvorgang in den Bügel eingesetzt oder von diesem entnommen werden kann, um die zuvor erwähnten Reinigungs-, Sterilisier- oder Austauschvorgänge durchzuführen.

In einer weiteren Ausgestaltung der Erfindung sind dazu in den zwei Haltearmen Aussparungen vorhanden, in die seitlich vorstehende Enden einer Lagerwelle der Rolle einclipsbar sind.

Diese Maßnahme hat den Vorteil, dass die zuvor erwähnten Vorteile besonders einfach erzielt werden können. Dies eröffnet beispielsweise auch die Möglichkeit, lediglich die Rolle als Wegwerfteil auszugestalten, wohingegen der Bügel und die Halterung als wieder verwendbares Teil ausgebildet sind, d.h. nach einem Gebrauch wird nur jeweils eine neue Rolle eingeclipst.

In einer weiteren Ausgestaltung der Erfindung ist die Umfangsfläche der Rolle mit einem reibungsstarken Material versehen.

Diese Maßnahme hat den Vorteil, dass zum einen der Kontakt zwischen dem Finger der meist durch einen Handschuh bedeckt ist und der Rolle intensiv ist und andererseits der Kontakt zwischen der Rolle und dem zu transportierenden Faden.

In einer weiteren Ausgestaltung der Erfindung ist in der Rolle eine umfängliche Nut vorhanden, in der der Faden aufnehmbar ist.

Diese Maßnahme hat den Vorteil, dass durch die Nut in der Rolle zusätzlich noch für eine Führung des Fadens gesorgt wird.

In einer weiteren Ausgestaltung der Erfindung ist die Nut zwischen zwei O-Ringen ausgebildet, die auf der Umfangsfläche aufgebracht sind.

Diese Maßnahme hat den Vorteil, dass durch die O-Ringe das reibungsstarke Material bereitgestellt werden kann und zugleich zwischen diesen eine Nut zur Führung des Fadens ausgebildet ist. Ferner wird verhindert, dass die Fäden gequetscht werden und dadurch nicht mehr durch den Handgriff und die Nadel zu führen sind.

In einer weiteren Ausgestaltung der Erfindung ist der Bügel schwenkbar an der Halterung angebracht.

Diese Maßnahme hat den Vorteil, dass durch die Schwenkbarkeit von der Handhabungsperson ein mehr oder weniger starker Druck auf den Faden aufgeübt werden kann, je nachdem wie das von dieser Handhabungsperson gewünscht wird.

Das Verschwenken erleichtert auch die Handhabung, beispielsweise beim Einfädeln des Fadens oder beim Anlegen oder Abnehmen der Vorrichtung.

In einer weiteren Ausgestaltung der Erfindung ist der schwenkbare Bügel gegen die Kraft einer Feder verschwenkbar.

Diese Maßnahme hat den Vorteil, dass die Handhabungsperson den Bügel gegen die Kraft einer Feder auf den Faden drückt, was ein besseres Kontaktgefühl für die Handhabungsperson gibt. Zugleich kann die Kraft der Feder dazu herangezogen werden, dass sich der Bügel automatisch hochstellt, d.h. von dem Faden abhebt, so dass dann entsprechende Manipulationen wie beispielsweise das Einfädeln oder dergleichen durchgeführt werden kann, ohne dass die Rolle von Hand vom Faden abgehoben werden muss.

In einer weiteren Ausgestaltung der Erfindung ist der schwenkbare Bügel über eine Raste mit der Halterung feststellbar.

Diese Maßnahme hat den Vorteil, dass die Raste den Bügel in einer auf den Handgriff hin verschwenkten Position hält.

In einer weiteren Ausgestaltung der Erfindung weist die Halterung eine Drehsicherung auf.

Dies ist insbesondere von Vorteil, wenn die Vorrichtung auf einen stabförmigen Körper mit etwa rundem Querschnitt aufgeclipst wird. Die Verdrehsicherung sorgt dafür, dass während der Handhabung sich die Vorrichtung nicht verdreht.

In einer weiteren Ausgestaltung der Erfindung ist die Drehsicherung als von der Halterung vorstehende Vorsprünge ausgebildet.

Diese Maßnahme hat den Vorteil, dass die Verdrehsicherung durch einfache Bauteile bewerkstelligt werden kann, die beispielsweise schon beim originären Herstellungsvorgang, beispielsweise beim Spritzen als Kunststoffteil mit vorgesehen werden können.

In einer weiteren Ausgestaltung der Erfindung kann die erfindungsgemäße Vorrichtung als Set mit einem chirurgischen Nahtinstrument verbunden, angeboten oder verkauft werden, wobei das chirurgische Nahtinstrument einen etwa stabförmigen Körper aufweist, durch den ein chirurgischer Faden von proximal nach distal zu einem Nahtaufsatz führbar ist, mit einer Mulde im Körper, längs der ein Abschnitt des Fadens freiliegend ist, wobei die erfindungsgemäße Vorrichtung so angebracht ist, dass die Rolle auf dem freiliegenden Abschnitt des Fadens in der Mulde zum Liegen kommt.

In diesem Set wird nun sowohl das chirurgische Nahtinstrument als auch die anclipsbare Vorrichtung angeboten, so dass alle Varianten durchführbar sind, also der Betrieb mit oder ohne der angeclipsten Vorrichtung.

Es versteht sich, dass die vorstehend genannten und die nachstehend noch zu erläuternden Merkmale nicht nur in der jeweils angegebenen Kombination, sondern auch in anderen Kombinationen einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Ansicht eines ersten Ausführungsbeispiels einer erfindungsgemäßen Vorrichtung, bei der der Bügel gegen die Kraft einer Feder aufstellbar ist,
- Fig. 2: die Vorrichtung von Fig. 1, wobei der verschwenkbare Bügel in einer verrasteten Endstellung dargestellt ist,
- Fig. 3: eine Seitenansicht eines chirurgischen Nahtinstrumentes, an das die Vorrichtung von Fig. 1 und 2 anclipsbar ist,
- Fig. 4: eine der Fig. 3 entsprechende Seitenansicht, wobei die Vorrichtung von Fig. 1 und 2 angeclipst ist,
- Fig. 5: eine Draufsicht auf den Zusammenbau von Fig. 4,
- Fig. 6: eine weitere Ausführungsvariante mit einer Verdrehsicherung,
- Fig. 7: eine Seitenansicht einer weiteren Ausführungsform einer erfindungsgemäßen Vorrichtung mit einem verschwenkbaren Bügel, allerdings ohne Verschwenkbarkeit gegen die Kraft einer Feder,
- Fig. 8: eine perspektivische Ansicht der Vorrichtung von Fig. 7,
- Fig. 9: eine der Darstellung von Fig. 7 entsprechende Seitenansicht einer weiteren Ausführungsvariante mit starrem, also nicht verschwenkbarem Bügel, und
- Fig. 10: eine perspektivische Ansicht der Ausführungsform von Fig. 9.

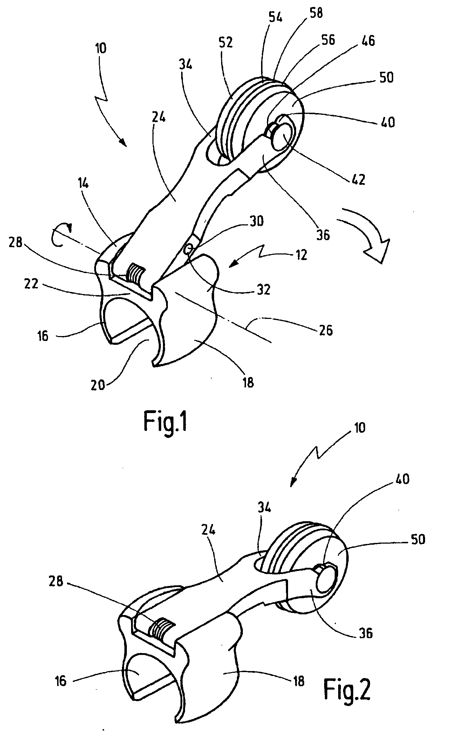

Ein in Fig. 1 und 2 dargestelltes erstes Ausführungsbeispiel einer erfindungsgemäßen Vorrichtung ist in ihrer Gesamtheit mit der Bezugsziffer 10 bezeichnet.

Die Vorrichtung 10 weist eine Halterung 12 in Form eines U-förmigen Klemmstückes 14 auf. Das Klemmstück 14 weist zwei vorstehende gebogene Schenkel 16 und 18 auf, die eine Öffnung 20 umgrenzen. Die beiden Schenkel 16 und 18 sind so ausgebildet, dass sie unter kurzfristigem Aufspreizen auf einen Körper aufgeclipst werden können, der in etwa die Form der Öffnung 20 belegt und die diesen Körper fest umschließen, nachdem die beiden Schenkel bzw. das Klemmstück 14 auf diesen Körper aufgeclipst worden ist.

An einem Querstück 22, das die beiden Schenkel 16 und 18 miteinander verbindet, ist schwenkbar ein Bügel 24 angebracht. Der Bügel 24 ist über einen hier nicht näher ersichtlichen Achsstift montiert, so dass er um eine Achse 26 verschwenkbar ist.

Um den Achsstift ist eine Feder 28 gelegt, die so vorgespannt ist, dass sie den Bügel 24 in die in Fig. 1 dargestellte Ausrichtung aufrichtet. Dazu stützt sich ein Schenkel der Feder 28 auf dem Querstück 22 ab.

Der Bügel 24 kann somit gegen die Kraft der Feder 28 um die Achse 26 verschwenkt werden, beispielsweise in die in Fig. 2 dargestellte Position. Aus Fig. 1 ist zu entnehmen, dass eine Raste 30 vorgesehen ist, um den Bügel 24 in der in Fig. 2 dargestellten verrasteten Position zu halten. Die Raste 30 weist eine Kugelraste 32 auf, wobei in Fig. 1 die Kugel der Kugelraste ersichtlich ist. An der Innenseite des oberen Endes des Schenkels 18 ist eine entsprechende Aussparung vorhanden, in die die Kugel 32 einrasten kann. Diese Situation ist in Fig. 2 dargestellt, d.h. in dieser Situation ist der Bügel 24 verrastet. Um den Bügel wieder in die in Fig. 1 gezeigte Darstellung zu bringen muss zunächst die Kraft der Kugelraste überwunden werden, anschließend stellt die Kraft der Feder 28 den Bügel 24 auf.

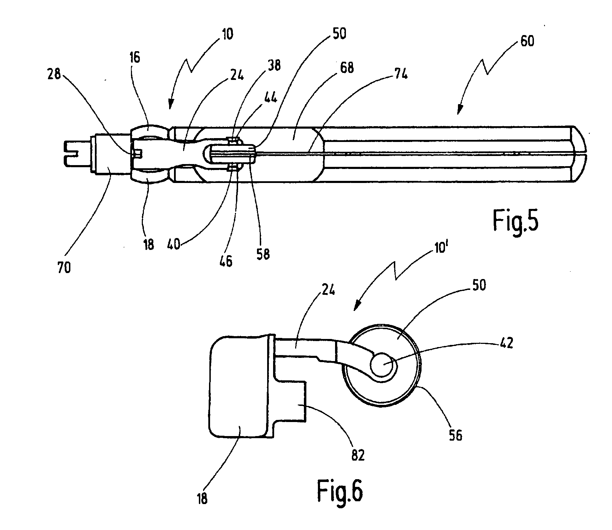

An dem der Achse 26 gegenüberliegenden Ende weist der Bügel 24 zwei gabelartig vorspringende Haltearme 34 und 36 auf, die jeweils eine Aussparung 38 bzw. 40 aufweisen. In die Aussparungen 38 und 40 können eine Lagerwelle 42 bzw. deren seitlich vorstehende Enden 44 und 46 (siehe Fig. 5) eingedrückt werden. Die Lagerwelle 42 trägt eine Rolle 50. Die Rolle 50 ist frei drehbar um die Lagerwelle 42.

Eine Umfangsfläche 52 der Rolle ist mit zwei im Abstand voneinander angeordneten Silicon-O-Ringen 54 und 56 bestückt, so dass zwischen diesen eine Nut 58 ausgebildet ist.

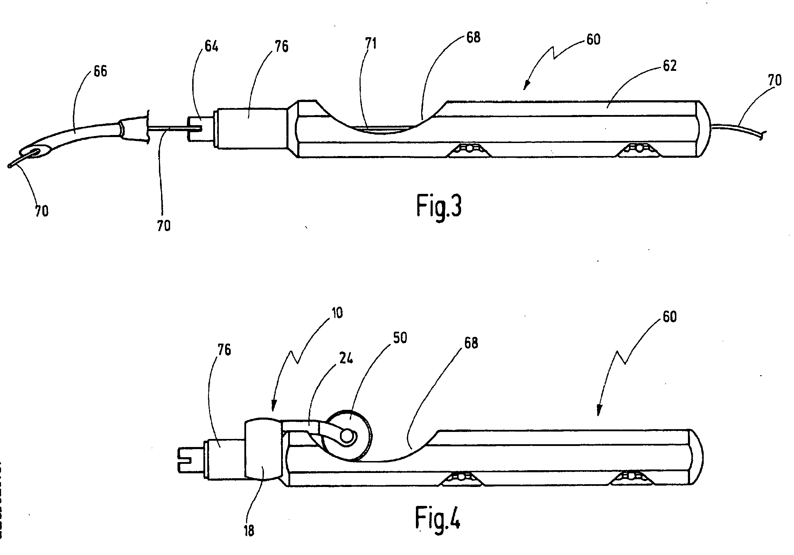

In den Fig. 3 bis 5 ist ein Anwendungsbereich der Vorrichtung 10 dargestellt, nämlich die Verwendung in Zusammenhang mit einer chirurgischen Nahtvorrichtung 60, wie sie in Fig. 3 dargestellt ist. Eine solche chirurgische Nahtvorrichtung, wie sie in Fig. 3 gezeigt ist, wird beispielsweise von der Anmelderin unter der Produktbezeichnung 28179 HG vertrieben.

Diese chirurgische Nahtvorrichtung 60 weist einen stabförmigen Körper 62 auf, der als längserstreckter Handgriff ausgebildet ist. Am distalen Ende 64 können unterschiedliche Nahtaufsätze 66 aufgesetzt werden, je nachdem, was für ein Nähvorgang durchgeführt werden soll. Im Bereich dieses Endes ist im Körper 62 eine Mulde 68 ausgespart, die als eine Art Fingermulde ausgebildet ist.

Durch den stabförmigen Körper 62 hindurch kann ein chirurgischer Faden 70 von proximal nach distal durchgefädelt werden, der dann auch durch den Nahtaufsatz 66 hindurchgefädelt ist, über den dann der Nähvorgang bewerkstelligt wird. Aus der Seitenansicht von Fig. 3 ist zu entnehmen, dass ein Abschnitt 71 des chirurgischen Fadens 70 in der Mulde 68 freiliegend ist.

Die chirurgische Nahtvorrichtung 60 kann so betrieben werden, dass der stabförmige Körper 62 nach Durchfädeln des chirurgischen Fadens 70 und Aufsetzen eines entsprechend gewünschten Nahtaufsatzes 66 mit einer Hand so ergriffen wird, dass beispielsweise der Daumen der Hand im Bereich der Mulde 68 zum Liegen kommt. Wird nun der Daumen auf den Abschnitt 71 des Fadens 70 gelegt und hin- und herbewegt, kann dadurch der Faden 70 hin- und herbewegt werden.

Um diesen Vorgang zu erleichtern und zu unterstützen ist, wie in Fig. 4 dargestellt, die aus Fig. 1 und 2 ersichtliche Vorrichtung auf einen zylindrischen Fortsatz 76 der chirurgischen Nahtvorrichtung 60 aufgeclipst, und zwar derart, dass die Rolle 50 auf dem Abschnitt 71 des chirurgischen Fadens 70 zum Liegen kommt, der die Mulde 68 durchquert.

Die Handhabung des Zusammenbaus von Fig. 4 ist gleich wie zuvor im Zusammenhang mit Fig. 3 beschrieben, d.h. es wird ein Faden 70 durchgefädelt und ein entsprechender Nahtaufsatz 66 angesetzt, wobei hier zum Unterschied bei der Ausgestaltung von Fig. 3 nunmehr der Daumen der Handhabungsperson auf die Rolle 50 gelegt wird und mittels der Rolle 50 der Faden hin- und herbewegt wird.

Der Faden 70 liegt in der Nut 58 zwischen den beiden Silicon-O-Ringen 54 und 56, so dass ein intensiver Kontakt vorhanden ist. Die Position des Bügels 24 von Fig. 4 entspricht nun der Position von Fig. 2, d.h. der Bügel 24 ist verrastet und kann nicht weiter in die Mulde 68 hineingedrückt werden.

Wird die Kraft der Verrastung überwunden, stellt sich der Bügel 24 hoch, so dass dann beispielsweise die Vorrichtung 10 unter Ergreifen des Bügels wieder von dem zylindrischen Fortsatz 76 abgezogen oder umgekehrt beispielsweise in dieser Position auf diesen aufgeclipst werden kann.

Aus der Draufsicht von Fig. 5 ist zu erkennen, dass der Faden 70 in einer Rille 74 geführt ist, die mit der Nut 58 ausgerichtet ist. Ist beispielsweise ein Faden zu verarbeiten, der eine extrem glatte Außenfläche hat, und ist aufgrund des Operationsvorgangs nicht auszuschließen, dass die Hand bzw. der Latexhandschuh feucht ist, kann durch Aufsetzen der Vorrichtung 10 die Handhabung des Fadens, also das Hin- und Herschieben wesentlich erleichtert und vor allem mit einem wesentlich besseren Handhabungsgefühl behandelt werden.

Nach dem Handhabungsvorgang kann die Vorrichtung 10 wieder abgezogen werden.

Zum Reinigen kann die Rolle 50 aus den Aussparungen 38 und 40 der Haltearme 44, 36 nach oben ausgedrückt werden, so dass dann eine Reinigung einfach erfolgen kann. Es kann auch vorgesehen sein, die Rolle 50 als Wegwerfteil auszubilden, so dass dann für einen weiteren Bearbeitungsvorgang eine andere neue Rolle 50 eingeclipst wird.

Dies eröffnet auch die Möglichkeit, bei unterschiedlich ausgestalteten Fäden unterschiedlich ausgestaltete Rollen 50 aufzusetzen, insbesondere mit unterschiedlicher Ausgestaltung der Umfangsfläche, wie das nachfolgend noch beschrieben wird.

In Fig. 6 ist eine weitere Variante einer Vorrichtung 10' dargestellt, die in ihren prinzipiellen Bauteilen gleich ausgebildet ist, wie die Fig. 1 und 2 dargestellte Vorrichtung 10, so dass daher für diese gleichen Bauteile auch die gleichen Bezugszeichen verwendet sind.

Im Unterschied zu der in Fig. 1 und 2 dargestellten Vorrichtung 10 weist die Vorrichtung 10' jeweils von den Schenkeln 16 bzw. 18 in Richtung der Rolle 50 vorstehende Vorsprünge 82 auf, die eine Drehsicherung darstellen, wenn die Vorrichtung 10' auf einen stabförmigen Körper 62 aufgeclipst ist, der ein relativ rundes Profil aufweist.

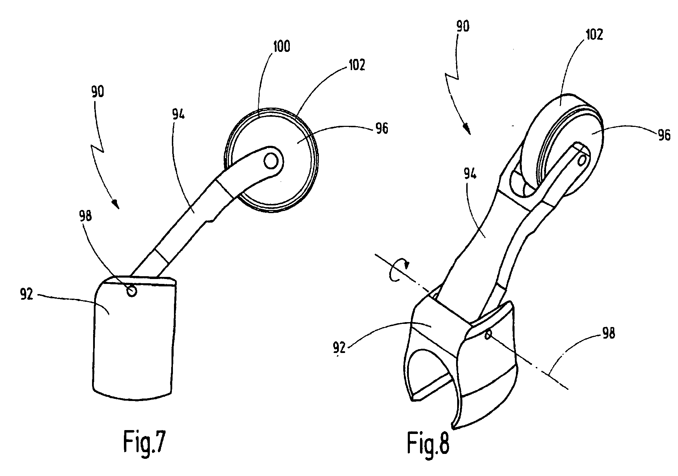

Bei der in Fig. 7 und 8 dargestellten weiteren Ausgestaltung einer erfindungsgemäßen Vorrichtung 90 weist diese ebenfalls, wie in Zusammenhang mit Fig. 1 und 2 beschrieben, eine Halterung 92 auf, an der ein Bügel 94 um eine Achse 98 schwenkbar befestigt ist. Auch hier trägt der Bügel 94 eine Rolle 96.

Im Gegensatz zu der in Fig. 1 und 2 dargestellten Vorrichtung 10 ist hier der Bügel 94 frei schwenkbar und nicht gegen die Kraft einer Feder. Hier ist auch keine Raste vorgesehen, so dass hier der Handhabungsperson freisteht, den Bügel 94 bzw. die Rolle 96 mit einer von ihm gewünschten Kraft auf den Faden aufzudrücken.

Diese Variante wird einzusetzen sein, wenn die Handhabungsperson genau diese Vorgehensweisen wünscht, also sie selbst bestimmen möchte, mit welchem Druck sie die Rolle 96 auf den Faden aufdrückt.

Auch die Vorrichtung von Fig. 7 und 8 kann auf eine in Fig. 3 dargestellte Vorrichtung entsprechend aufgeclipst werden. Aus Fig. 8 ist zu entnehmen, dass die Umfangsfläche 100 breitflächig mit einem reibungsstarken Belag 102 versehen ist, beispielsweise einem Band aus einem Siliconkautschuk.

Wie aus der Draufsicht von Fig. 5 zu entnehmen ist, ist der Faden 70 in einer Rille 74 geführt, so dass auch ohne eine vorhandene Nut an der Umfangsfläche der Rolle 96 eine relativ seitlich verschiebfreie Hin- und Herbewegung des Fadens möglich ist, nämlich wenn das umfängliche Band an dem reibungsstarkem Belag 102 großflächig auf den Faden 70 gedrückt wird.

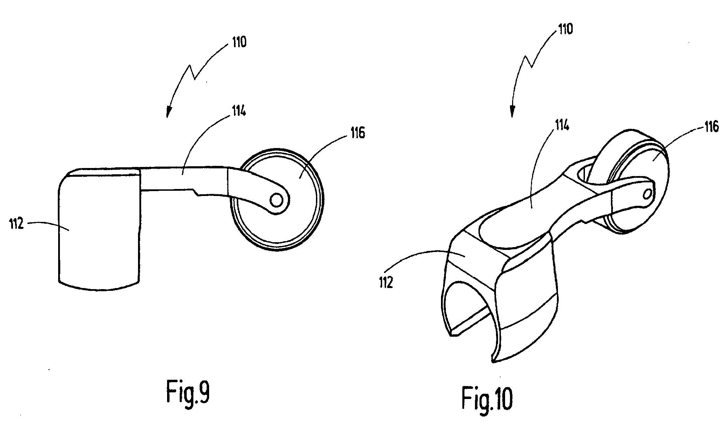

In den Fig. 9 und 10 ist eine weitere, vereinfachtere Variante einer erfindungsgemäßen Vorrichtung 110 dargestellt. Auch die Vorrichtung 110 weist eine wie zuvor beschriebene Halterung 112 auf, an der allerdings starr und unbeweglich ein Bügel 114 angebracht ist, der eine Rolle 116 trägt. Auch die Vorrichtung 110 kann beispielsweise auf die chirurgische Nahtvorrichtung von Fig. 3 aufgeclipst werden. Da hier ja der Bügel 114 nicht verschwenkbar ist, muss die Geometrie von Halterung 112 und Bügel 114 sowie die Geometrie der Rolle 116 so auf die chirurgische Nahtvorrichtung 60 abgestimmt sein, dass nach dem Aufclipsen sichergestellt ist, dass die Umfangsfläche der Rolle 116 auf dem Faden aufliegt.

Wenn also beispielsweise die Vorrichtung von Fig. 9 und 10 als Set zusammen mit der Vorrichtung von Fig. 3 angeboten wird, können schon herstellerseits entsprechend Maßnahmen getroffen werden, dass die zuvor bedingten Erfüllungen jeweils gegeben sind.

In diesem Fall ist dann die Vorrichtung 110 als einfaches Kunststoffspritzteil anzufertigen, indem lediglich noch die Rolle 116 eingesetzt werden muss.

Dies eröffnet die Möglichkeit, die gesamte Vorrichtung 110 als Einmal- bzw. Wegwerfteil herzustellen.

## Patentansprüche

1. Vorrichtung zum Bewegen eines Fadens (70) eines chirurgischen Nahtinstrumentes (60), wobei das Nahtinstrument (60) einen etwa stabförmigen Körper (62) aufweist, durch den ein chirurgischer Faden (70) von proximal nach distal zu einem distalen Nahtaufsatz (66) führbar ist, wobei der Faden (70) in einer Mulde (68) des Körpers (62) freiliegt, so dass der freiliegende Abschnitt (71) des Fadens (70) unmittelbar durch einen Finger einer Hand einer Handhabungsperson ohne Zuhilfenahme einer Rolle, bewegbar ist, wobei die Vorrichtung einen Bügel (24, 94, 114) aufweist, der an einem Ende eine frei drehbare Rolle (50, 96, 116) trägt und ferner eine Halterung (12, 92, 112) aufweist, über die der Bügel (24, 94, 114) derart an das stabförmige Nahtinstrument (60) lösbar anclipsbar ist, dass die Rolle (50, 96, 116) im Bereich der Mulde (68) des etwa stabförmigen Körpers (62) auf dem freiliegenden Fadenabschnitt (71) zum Liegen kommt, so dass der Faden (70) durch die Handhabungsperson über der Rolle (50, 96, 116) bewegbar ist.

2. Vorrichtung nach Anspruch 1, **dadurch gekennzeichnet, dass** die Rolle (50) abnehmbar am Bügel (24) angebracht ist.

3. Vorrichtung nach Anspruch 1 oder 2, **dadurch gekennzeichnet, dass** die Rolle (50) in zwei Haltearmen (34, 36) des Bügels (24) gehalten ist.

4. Vorrichtung nach einem der Ansprüche 1 bis 3, **dadurch gekennzeichnet, dass** die Rolle (50) in den Bügel (24) einclipsbar ist.

5. Vorrichtung nach Anspruch 3 oder nach Anspruch 3 und 4, **dadurch gekennzeichnet, dass** in den zwei Haltearmen (34, 36) Aussparungen (38, 40) vorhanden sind, in die seitlich vorstehende Enden (44, 46) einer Lagerwelle (42) der Rolle (50) einclipsbar sind.

6. Vorrichtung nach einem der Ansprüche 1 bis 5, **dadurch gekennzeichnet, dass** die Umfangsfläche (52, 100) der Rolle (50, 96, 116) mit einem reibungsstarken Material versehen ist.

7. Vorrichtung nach einem der Ansprüche 1 bis 6, **dadurch gekennzeichnet, dass** in der Rolle (50) eine umfängliche Nut (58) vorhanden ist, in der der Faden (70) aufnehmbar ist.

8. Vorrichtung nach Anspruch 7, **dadurch gekennzeichnet, dass** die Nut (58) zwischen zwei O-Ringen (54, 56) auf der Umfangsfläche (52) ausgebildet ist.

9. Vorrichtung nach einem der Ansprüche 1 bis 8, **dadurch gekennzeichnet, dass** der Bügel (24, 94) schwenkbar an der Halterung (12, 92) angebracht ist.

10. Vorrichtung nach Anspruch 9, **dadurch gekennzeichnet, dass** der schwenkbare Bügel (24) gegen die Kraft einer Feder (28) verschwenkbar ist.

11. Vorrichtung nach Anspruch 9 oder 10, **dadurch gekennzeichnet, dass** der schwenkbare Bügel (24) über eine Raste (30) mit der Halterung (12) feststellbar ist.

12. Vorrichtung nach einem der Ansprüche 1 bis 11, **dadurch gekennzeichnet, dass** die Halterung (12) eine Drehsicherung (82) aufweist.

13. Vorrichtung nach Anspruch 12, **dadurch gekennzeichnet, dass** die Drehsicherung durch von der Halterung (12) vorstehende Vorsprünge (82) ausgebildet ist.

14. Chirurgisches Nahtinstrument (60), mit einem etwa stabförmigen Körper (62), durch den ein chirurgischer Faden (70) von proximal nach distal zu einem Nahtaufsatz (66) führbar ist, mit einer Mulde (68) im Körper (62), längs der ein Abschnitt (71) des Fadens (70) freiliegend geführt ist, so dass dieser unmittelbar durch einen Finger einer Hand einer Handhabungsperson, ohne Zuhilfenahme einer Rolle, bewegbar ist, **dadurch gekennzeichnet, dass** am Körper (62) eine Vorrichtung (10, 10', 90, 110) nach einem der Ansprüche 1 bis 13 derart angeclipst ist, dass die Rolle (50) auf dem freiliegenden Abschnitt (71) des Fadens (70) in der Mulde (68) zum Liegen kommt, so dass der Faden (70) durch die Handhabungsperson über die Rolle (50, 96, 116) bewegbar ist.

## Claims

1. Device for moving a thread (70) of a surgical sewing instrument (60), wherein the sewing instrument (60) has an approximately rod-like body (62), through which a surgical thread (70) can be guided from proximal to distal to a distal sewing attachment (66), wherein the thread (70) is exposed in a cavity (68) of the body (62), with the result that the exposed section (71) of the thread (70) can be moved directly with a finger of a hand during handling without the aid of a roller, wherein the device has a bail (24, 94, 114) supporting on one end a roller (50, 96, 116) which can be rotated freely, and further has a holder (12, 92, 112), via which the bail (24, 94, 114) can be clipped onto the rod-like sewing instrument (60) in that the roller (50, 96, 116) comes to a rest on the exposed thread section (71) in the area of the cavity (68) of the rod-like body (62), with the result that the thread (70) can be moved by an operator via the roller (50, 96, 116).

2. Device of claim 1, **characterized in that** the roller (50) is attached removably to the bail (24).

3. Device of claims 1 or 2, **characterized in that** the roller (50) is held in two retaining arms (34, 36) of the bail (24).

4. Device of any one of claims 1 through 3, **characterized in that** the roller (50) can be clipped into the bail (24).

5. Device of claims 3 or claims 3 and 4, **characterized in that** there are recesses (38, 40) in the two retaining arms (34, 36), into which laterally projecting ends (44, 46) of a bearing shaft (42) of the roller (50) can be clipped.

6. Device of any one of claims 1 through 5, **characterized in that** in the peripheral surface (52, 100) of the roller (50, 96, 116) is provided with a friction-fast material.

7. Device of any one of claims 1 through 6, **characterized in that** there is a circumferential groove (58) in the roller (50), in which the thread (70) can be taken up.

8. Device of claim 7, **characterized in that** the groove (58) is designed between two O-rings (54, 56) on the peripheral surface (52).

9. Device of any one of claims 1 through 8, **characterized in that** the bail (24, 94) is attached pivotably on the holder (12, 92).

10. Device of claim 9, **characterized in that** the pivotable bail (24) can be pivoted against the force of a spring (28).

11. Device of claims 9 or 10, **characterized in that** the pivotable bail (24) can be fixed by a catch (30) to the holder (12).

12. Device of any one of claims 1 through 11, **characterized in that** the holder (12) has a rotation safety device (82).

13. Device of claim 12, **characterized in that** the rotation safety device is designed by projections (82) standing out from the holder (12).

14. Surgical sewing instrument (60) having an approximately rod-like body (62), through which a surgical thread (70) can be guided from proximal to distal to a sewing attachment (66), having a cavity (68) within the body (62) along which a section (71) of the thread (70) is guided in an exposed manner, in that it can be directly moved by a finger of a hand of an operator, without the aid of a roller, **characterized in that** a device (10, 10', 90, 110) of any one of claims 1 through 13 is clipped onto the body (62) **in that** the roller (50) comes to a rest on the exposed section (71) of the thread (70) in the cavity, with the result that the thread (70) can be moved by an operator via the roller (50, 96, 116).

## Revendications

1. Dispositif pour déplacer un fil (70) d'un instrument chirurgical de suture (60), l'instrument de suture (60) présentant un corps (62) approximativement en forme de barre, à travers lequel un fil chirurgical (70) peut être guidé d'une direction proximale dans une direction distale jusqu'à un embout de suture distal (66), le fil (70) étant exposé dans un creux (68) du corps (62), de telle sorte que la portion exposée (71) du fil (70) puisse être déplacée directement par un doigt d'une main d'un opérateur sans devoir utiliser de bobine, le dispositif présentant un étrier (24, 94, 114) qui supporte à une extrémité une bobine librement rotative (50, 96, 116) et présente en autre une fixation (12, 92, 112), par le biais de laquelle l'étrier (24, 94, 114) peut être enclipsé de manière desserrable sur l'instrument de suture (60) en forme de barre de telle sorte que la bobine (50, 96, 116) dans la région du creux (68) du corps (62) approximativement en forme de barre vienne s'appliquer sur la portion de fil exposée (71), de telle sorte que le fil (70) puisse être déplacé par l'opérateur par le biais de la bobine (50, 96, 116).

2. Dispositif selon la revendication 1, **caractérisé en ce que** la bobine (50) est montée de manière amovible sur l'étrier (24).

3. Dispositif selon la revendication 1 ou 2, **caractérisé en ce que** la bobine (50) est retenue dans deux bras de retenue (34, 36) de l'étrier (24).

4. Dispositif selon l'une quelconque des revendications 1 à 3, **caractérisé en ce que** la bobine (50) peut être enclipsée dans l'étrier (24).

5. Dispositif selon la revendication 3 ou selon la revendication 3 et la revendication 4, **caractérisé en ce que** dans les deux bras de retenue (34, 36) sont prévus des évidements (38, 40) dans lesquels des extrémités saillant latéralement (44, 46) d'un arbre de palier (42) de la bobine (50) peuvent être enclipsées.

6. Dispositif selon l'une quelconque des revendications 1 à 5, **caractérisé en ce que** la surface périphérique (52, 100) de la bobine (50, 96, 116) est pourvue d'un matériau de forte friction.

7. Dispositif selon l'une quelconque des revendications 1 à 6, **caractérisé en ce que** dans la bobine (50) est prévue une rainure périphérique (58), dans laquelle peut être reçu le fil (70).

8. Dispositif selon la revendication 7, **caractérisé en ce que** la rainure (58) est réalisée entre deux joints toriques (54, 56) sur la surface périphérique (52).

9. Dispositif selon l'une quelconque des revendications 1 à 8, **caractérisé en ce que** l'étrier (24, 94) est monté de manière pivotante sur la fixation (12, 92).

10. Dispositif selon la revendication 9, **caractérisé en ce que** l'étrier pivotant (24) peut pivoter à l'encontre de la force d'un ressort (28).

11. Dispositif selon la revendication 9 ou 10, **caractérisé en ce que** l'étrier pivotant (24) peut être fixé par le biais d'un cliquet (30) à la fixation (12).

12. Dispositif selon l'une quelconque des revendications 1 à 11, **caractérisé en ce que** la fixation (12) présente une fixation antirotation (82).

13. Dispositif selon la revendication 12, **caractérisé en ce que** la fixation antirotation est réalisée par des saillies (82) faisant saillie depuis la fixation (12).

14. Instrument chirurgical de suture (60), comprenant un corps (62) approximativement en forme de barre, à travers lequel un fil chirurgical (70) peut être guidé d'une direction proximale dans une direction distale jusqu'à un embout de suture (66), comprenant un creux (68) dans le corps (62), le long duquel une portion (71) du fil (70) est guidée de manière exposée, de telle sorte que celle-ci puisse être déplacée directement par un doigt d'une main d'un opérateur sans devoir utiliser de bobine, **caractérisé en ce qu'**un dispositif (10, 10', 90, 110) selon l'une quelconque des revendications 1 à 13 est enclipsé sur le corps (62) de telle sorte que la bobine (50) vienne s'appliquer sur la portion exposée (71) du fil (70) dans le creux (68), de telle sorte que le fil (70) puisse être déplacé par l'opérateur par le biais de la bobine (50, 96, 116).
